Europäisches Patentamt

**European Patent Office**

Office européen des brevets

(11) Numéro de publication: **0 122 841**
**A1**

## (12) DEMANDE DE BREVET EUROPEEN

(21) Numéro de dépôt: 84400648.6

(22) Date de dépôt: 30.03.84

(51) Int. Cl.³: **C 12 P 21/00**
C 12 N 5/00, C 12 N 15/00
G 01 N 33/58

(30) Priorité: 31.03.83 FR 8305384

(43) Date de publication de la demande:
24.10.84 Bulletin 84/43

(84) Etats contractants désignés:
AT BE CH DE FR GB IT LI LU NL SE

(71) Demandeur: INSTITUT PASTEUR
25-28, rue du Docteur Roux
F-75724 Paris Cedex 15(FR)

(71) Demandeur: INSTITUT NATIONAL DE LA SANTE ET DE
LA RECHERCHE MEDICALE (INSERM)
101, rue de Tolbiac
F-75654 Paris Cedex 13(FR)

(72) Inventeur: Horaud, Florian
2, rue Albert de Mun
F-92190 Meudon(FR)

(72) Inventeur: Boue, André
7, rue du Docteur Vaillant
F-78210 Saint Cyr L'Ecole(FR)

(72) Inventeur: Amadei, Claire
9, rue Bernouilli
F-75008 Paris(FR)

(72) Inventeur: Michelson, Susan
11, bis rue Pauline Borghèse
F-92200 Neuilly sur Seine(FR)

(74) Mandataire: Gutmann, Ernest
Cabinet Plasseraud 84, rue d'Amsterdam
F-75009 Paris(FR)

(54) Anticorps monoclonaux anti-cytomégalovirus humains, hybridomes secréteurs de ces anticorps et polypeptides porteurs d'un déterminant antigénique séquentiel de cytomégalovirus humains.

㊗ L'invention concerne un anticorps monoclonal-anticytomégalovirus humain, caractérisé par ses capacités:

à neutraliser les virus de HCMV;

à donner lieu à des réactions détectables par immunofluorescence, sur des cellules de cultures d'origine humaine préalablement infectées avec HCMV et fixées à l'acétone;

à réagir avec essentiellement un seul polypeptide viral d'un poids moléculaire de l'ordre de 94 000, porteur d'un épitope séquentiel continu et susceptible d'être isolé à partir du cytoplasme de cellules d'origine humaine préalablement infectées par HCMV en la présence d'inhibiteurs de protéase;

de préférence à réagir avec la protéine A.

Anticorps monoclonaux anti-cytomégalovirus humains, hybridomes secréteurs de ces anticorps et polypeptides porteurs d'un déterminant antigénique séquentiel de cytomégalovirus humains.

---

L'invention est relative à de nouveaux anticorps monoclonaux anti-cytomégalovirus humains (HCMV) et à des hybridomes secréteurs de tels anticorps. Elle concerne plus particulièrement des anticorps de ce type qui soient capables de simultanément détecter les cytomégalovirus (CMV) humains, les cellules humaines infectées par le CMV et des polypeptides porteurs d'un déterminant antigénique séquentiel (continu) de CMV.

Il est connu que le CMV est la cause d'infections cliniques nombreuses s'étageant entre des manifestations infectieuses bénignes et des manifestations congénitales, par exemple des malformations, particulièrement sévères. Le CMV a également été reconnu comme cause de morbidité et mortalité chez les personnes ayant subi des transplantations d'organes, par exemple de rein . Le dépistage du CMV est un problème qui se pose donc de façon particulièrement aiguë. En outre la reconnaissance et l'isolement de polypeptides ou protéines vaccinants ou susceptibles d'être rendus vaccinants à l'égard du CMV ouvrirait des possibilités sérieuses de prévention desdites complications contre lesquelles on ne dispose à l'heure actuelle que de peu de moyens de traitement.

Divers auteurs ont déjà porté leur attention sur les techniques de diagnostic qui pourraient être basées sur l'utilisation d'anticorps monoclonaux à l'égard du cytomégalovirus humain. Les anticorps monoclonaux obtenus jusqu'à ce jour n'apportaient pas encore la solution attendue pour réaliser un diagnostic à la fois facile et fiable de l'infection aux cytomégalovirus.

En effet, il est connu que le CMV (membre de la famille des virus de l'herpès) induit dans les cellules infectées un nombre considérable de polypeptides, à divers stades du processus infectieux et à différents niveaux des cellules infectées. L.N. PEREIRA et Coll. ("Infection and Immunity", juin 1982, p. 924-932) rapportent qu'ils ont produit un grand nombre d'hybridomes secréteurs d'anticorps monoclonaux capables de reconnaître chez des cellules préalablement infectées et non fixées des polypeptides ou glycoprotéines induites par le CMV dans des cultures de ces cellules. Les différences observées par PEREIRA et Coll. au niveau des comportements relatifs des différents anticorps vis-à-vis du virus lui-même, des cellules infectées et des protéines glycoprotéines ou polypeptides susceptibles d'être isolés à partir d'extraits de ces cellules les ont conduits à formuler l'hypothèse, que la conformation des glycoprotéines virales reconnues par certains de ces anticorps à la surface membranaire des cellules infectées par les CMV, pourrait être différente de la conformation de l'enveloppe du virion. L'utilisation de certains des anticorps isolés pour réaliser des opérations de diagnostic du genre sus-indiqué a été évoquée par ces auteurs. Il résulte cependant de ce qui précède et des résultats expérimentaux fournis que les anticorps monoclonaux jugés les plus intéressants étaient également capables de précipiter à la fois plusieurs polypeptides. L'utilisation de ces anticorps pour reconnaître des déterminants antigéniques ou épitopes spécifiques ne pouvait donc être envisagée en raison du caractère non discriminant de la reconnaissance.

L.C. GOLDSTEIN et Coll. ("Infection and Immunity", octobre 1982, p. 273-281) ont également décrit un certain nombre d'anticorps monoclonaux susceptibles de reconnaître certains polypeptides localisés dans les noyaux ou dans des inclusions cytoplasmiques de cellules infectées par le CMV. Ces anticorps monoclonaux avaient

3

été sélectionnés par leur capacité à réagir avec des cellules infectées par le CMV, et préalablement fixées au méthanol absolu et séchées à l'air. Il ne résulte cependant pas de l'article que ces anticorps monoclonaux avaient un pouvoir neutralisant à l'égard des virus eux-mêmes ou encore étaient aptes à reconnaître des cellules infectées encore intactes.

L'invention a pour but de fournir des anticorps monoclonaux plus spécifiques encore que ceux qui ont été décrits dans l'état de la technique, dont l'utilisation dans des tests du diagnostic soit rendue plus aisée, notamment par la capacité qu'ils doivent posséder de neutraliser le virus entier et de reconnaître des cellules infectées encore intactes, et en outre de fournir un moyen de reconnaissance et d'isolement de polypeptides, protéines ou glycoprotéines porteurs d'un épitope ou déterminant antigénique séquentiel continu, c'est-à-dire d'un déterminant antigénique dont la reconnaissance par les anticorps ne soit plus liée à la conformation particulière de ce site sur les polypeptides, protéines ou glycoprotéines naturels du virus et dont la production par la cellule est induite par le virus qui l'infecte. L'invention résulte de la découverte qu'ont faite les inventeurs d'un hybridome secréteur d'anticorps monoclonaux répondant à l'ensemble de ces conditions et qui, en outre, est capable de reconnaître de façon sélective un déterminant antigénique séquentiel porté par une protéine ou glycoprotéine cytoplasmique dont le poids moléculaire avoisine 94.000, lorsqu'elle peut être isolée sans dégradation d'extraits cytoplasmiques de cellules infectées par une souche CMV, notamment par la souche Ad-169.

En d'autres termes, l'anticorps monoclonal anti-cytomégalovirus humain selon l'invention peut être défini par la combinaison de ses capacités :

- à neutraliser le virus de HCMV ;
- à donner lieu à des réactions détectables par immunofluorescence, sur des cellules de cultures d'origine humaine, préalablement infectées avec HCMV et fixées à l'acétone ;
- à réagir avec essentiellement un seul polypeptide viral d'un poids moléculaire de l'ordre de 94.000, porteur d'un épitope séquentiel continu et susceptible d'être isolé à partir du cytoplasme de cellules d'origine humaine préalablement infectées par HCMV, de préférence en la présence d'inhibiteurs de protéase;
- de préférence, à réagir avec la protéine A.

Les anticorps monoclonaux préférés sont ceux qui ne réagissent pas avec des récepteurs non spécifiques d'IgG, et plus particulièrement encore ceux qui se caractérisent par leur capacité à neutraliser plusieurs souches distinctes de CMV, notamment les souches connues sous les désignations Ad-169, Towne et Davis. Un anticorps monoclonal préféré est celui qui est produit par l'hybridome déposé à la Collection Nationale des Cultures de Micro-organismes (C.N.C.M.) sous le n° I-225 le 31/03/1983.

L'invention concerne naturellement également les hybridomes secréteurs de tels anticorps monoclonaux et un procédé pour leur obtention et leur isolement. Ce procédé met en jeu des hybridomes secréteurs d'anticorps monoclonaux neutralisant les virus HCMV, préalablement formés entre des cellules de myélomes et des cellules spléniques d'un animal préalablement immunisé contre un HCMV, tels que la souche HCMV Ad-169, ce procédé étant plus particulièrement caractérisé en ce que :
- l'on fait réagir lesdits anticorps neutralisants avec des cellules humaines en cultures préalablement infectées avec un HCMV et préalablement fixées avec de l'acétone, puis séchées, de préférence à l'air,
- l'on effectue une première sélection de ceux des clones qui donnent lieu à une réaction de fixation, détectable par immunofluorescence sur lesdites cellules humaines après fixation de ces dernières à l'acétone et
- le cas échéant, on effectue une seconde sélection de ceux des anticorps monoclonaux retenus à l'issue de la première sélection, qui réagissent sélectivement avec une protéine virale induite dans le cytoplasme desdites cellules infectées et obtenue à partir de celui-ci, cette protéine présentant un poids moléculaire de l'ordre de 94.000, ou contenue dans une fraction polypeptidique obtenue à partir dudit cytoplasme après séparation préalable des protéines ou polypeptides ayant des poids moléculaires différents et
- l'on récupère l'anticorps sélectionné à partir des produits de réaction obtenus.

La susdite récupération peut être réalisée de toute façon en soi connue, par exemple par complexation préalable du produit de réaction formé avec une protéine A, notamment de *Staphylococcus Aureus* et la dissociation du complexe formé dans un tampon ionique adéquat et la récupération à partir du mélange de dissociation de l'anticorps monoclonal, par exemple en mettant en jeu les masses moléculaires différentes des constituants du complexe dissocié.

En ce qui concerne plus particulièrement les cellules humaines mises en jeu dans l'opération de réaction des anticorps susceptibles d'être produits par les hybridomes en question plus haut, on peut avoir recours à tout type de cellule susceptible de supporter la réplication du HCMV Des cellules dont l'utilisation est avantageuse sont constituées par des fibroblastes humains de poumon du type MRC-5.

L'utilisation de l'acétone pour la fixation des cellules peut se révéler particulièrement efficace, en ce qu'elle permet la détection d'un nombre d'hybridomes beaucoup plus important que celui qui peut être obtenu en mettant en oeuvre une fixation au méthanol, même si toutes les autres étapes du procédé d'obtention et d'isolement d'hybridomes secréteurs sont par ailleurs les mêmes. Le procédé d'isolement selon l'invention implique de préférence également, au niveau de la susdite première sélection des hybridomes secréteurs, des mises en contact des anticorps neutralisants produits avec des cellules infectées chez lesquelles le processus infectieux a été interrompu, notamment par la fixation à l'acétone, après des intervalles de temps respective-ment croissants, à compter du moment où l'infection a été réali-sée. Normalement, des hybridomes secréteurs des anticorps monoclonaux recherchés peuvent être isolés à partir de cultures cellulaires chez lesquelles l'infection a été interrompue de 6 à 48 heures, notamment de 15 à 24 heures après le début de l'infection cellulaire.

L'invention concerne également les polypeptides ayant un poids moléculaire pouvant atteindre une valeur de l'ordre de 94.000 et porteurs de l'épitope séquentiel contenu dans la protéine virale cytoplasmique qui est susceptible d'être obtenue à partir d'une culture de cellules humaines, notamment de fibroblastes humains de poumon, préalablement infectés avec une culture de HCMV, notamment de la souche Ad-169. Ce polypeptide peut être isolé du cytoplasme des cellules infectées. Si l'on a soin de réaliser cette extraction dans des conditions interdisant sa dégradation, notamment par inhibition en présence de protéases appropriées, ou à basse température, par exemple à 40°C, ou les deux à la fois, on obtient un polypeptide de poids moléculaire d'environ 94.000. Mais l'invention concerne aussi tout polypeptide de poids moléculaire plus faible, contenant également l'épitope séquentiel reconnu par les susdits anticorps monoclonaux . Il apparaîtra clairement au spécialiste qu'à partir de l'instant où l'on dispose de l'anticorps monoclonal selon l'invention, il devient possible d'envisager l'isolement à partir de la protéine ou glycoprotéine ayant le poids moléculaire susdit d'environ 94.000, de séquences peptidiques plus petites contenant le même déterminant antigénique, en ayant recours à des techniques en soi connues de découpage du peptide initial intact par des enzymes susceptibles de le couper en des sites spécifiques. A titre d'exemple de telles protéines, on peut mentionner l'enzyme de *Staphylococcus Aureus* V8, l'α-chymotrypsine, la "protéase de glande sous-maxillaire de souris" (mouse submaxillary gland protease) commercialisée par la société BOEHRINGER, la collagénase de *Vibrio alginolyticus chemovar iophagus* qui reconnaît spécifiquement les dipeptides Gly-Pro et Gly-Ala.

Il devient alors possible, à partir des peptides fragmentés de façon contrôlée à l'aide de telles enzymes de détecter ceux qui contiennent le site antigénique par réaction avec l'anticorps monoclonal.

L'anticorps monoclonal selon l'invention peut donc également être à la base de ce qui peut être considéré comme un procédé de purification d'un polypeptide protéine ou glycoprotéine, ou encore d'un fragment de ces derniers, contenant un déterminant antigénique séquentiel du HCMV à partir d'extraits cytoplasmiques ou de lysats de cultures cellulaires humaines, préalablement infectées par HCMV. Dans une forme préférée de ce procédé, on aura recours à un anticorps monoclonal du type de ceux qui ont été définis plus haut, fixé au préalable, par exemple au bromure de cyanogène sur un support solide, tel que le réseau d'agarose à réticulation tri-dimensionnelle, commercialisé sous la marque SEPHAROSE par la société suédoise PHARMACIA AG.

Le caractère séquentiel continu du déterminant antigénique des polypeptides selon l'invention se déduit de ce que le polypeptide le portant peut toujours être isolé à partir de l'extrait cytoplasmique de cellules infectées, même lorsque l'opération de séparation est effectuée en présence de détergents puissants comme le dodécylsulfate de sodium (SDS), le déoxycholate de sodium, ou du détergent commercialisé sous la marque TRITON X-100, ou encore du β-mercapto-éthanol. Les détergents susmentionnés sont en effet connus pour leur capacité à "défaire" ou à "déplier" une protéine. Tout déterminant antigénique "conformation-

8

nel" cesserait alors d'être reconnu par l'anticorps monoclonal, et ce surtout dans l'hypothèse où le "site conformationnel" mettait en jeu des séquences d'acides aminés
dont le voisinage dans la protéine naturelle ne résultait
que du rapprochement de séquences non directement liées
entre elles, du fait de la conformation initiale de ladite
protéine. Il résulte de ce qui précède que cette protéine
glycoprotéine ou polypeptide porteur dudit site antigénique est vraisemblablement également immunogène et par
conséquent apte à induire la production *in vivo* d'anticorps neutralisant le virus lui-même.

Si les anticorps selon l'invention permettent
de détecter spécifiquement la protéine de poids moléculaire d'environ 94.000 au sein d'un extrait cellulaire
protégé contre les dégradations enzymatiques, à l'exclusion de tout autre polypeptide, protéine ou glycoprotéine
appartenant à un groupe de poids moléculaire distinct, il
en résulte que ce polypeptide, protéine ou glycoprotéine
pourra lui-même également être utilisé pour réaliser la
sélection et l'isolement d'anticorps monoclonaux susceptibles d'être produits par des hybridomes obtenus par des
fusions cellulaires mettant en jeu initialement d'autres
types de myélomes, d'une part, et d'autres HCMV pour l'immunisation des cellules spléniques destinées à être fusionnées
avec lesdites cellules de myélome, d'autre part.

Des caractéristiques supplémentaires de l'invention apparaîtront encore au cours de la description qui
suit d'exemples de production d'hybridomes et d'isolement
de ceux d'entre eux qui sont aptes à produire des anticorps conformes à l'invention.

Les techniques qui ont été mises en jeu dans
la réalisation de ces exemples ont été réalisées selon les
modes opératoires suivants :

1) <u>Préparation des hybridomes</u>

Des souris sont immunisées par injection intrapéritonéale d'une suspension de cellules MRC-5 humaines

9

infectées et décongelées. Des cellules avaient été congelées 5 jours après leur infection avec une souche de HCMV Ad-169. Une injection de rappel est administrée à ces souris 3 ou 4 semaines plus tard. Les souris sont sacrifiées et les rates récupérées en vue de leur fusion cellulaire 3 jours après l'injection de rappel. Les cellules spléniques sont fusionnées avec des cellules de myélome $SP^2$/OAg 14 (souche de SCHULMANN et Coll., "Nature", 1978, 276, 269/270) et les hybridomes formés sont sélectionnés dans un milieu RPMI contenant de l'hypoxanthine (5 mM) et de l'azaserine (1 mM) selon la technique décrite par R. CRAINIC et Coll., "Develop. Biol. Standard.", 1982, 50, 229-234, en rapport avec la production d'hybridomes secréteurs d'anticorps monoclonaux contre le virus de la poliomyélite.

2) Sélection des hybridomes positifs.

Les surnageants des cultures d'hybridomes ainsi retenus ont été sélectionnés pour leur capacité à secréter des anticorps spécifiques contre HCMV,par le test de neutralisation mettant en jeu la réduction des nombres de plages et par immunofluorescence indirecte, à l'aide de préparations d'antigènes tardifs. Une série d'hybridomes positifs est clonée par la méthode des dilutions limites et les surnageants de ces clones sont ensuite testés de la même façon par fluorescence indirecte et neutralisation du virus lui-même.

3) Neutralisation du virus.

Le test de neutralisation par la réduction des nombres de plages est réalisé de la façon suivante : On mélange 0,2 ml d'une suspension de virus contenant approximativement 100 unités formant des plages (pfu)de HCMV avec 0,2 ml de surnageant d'hybridomes et 5 unités hémolytiques ($HU^{50}$)

de complément de cobaye (chaque fois dans un volume de 25 µl) par puits dans des plaques de Costar à 24 puits.

Après incubation pendant 2 heures à 37°C dans une atmosphère de $CO_2$, on ajoute dans chaque puits 0,6 ml ($10^5$ cellules) d'une suspension de fibroblastes humains MRC-5 (JACOBS et Coll., "Nature", 1970, 277, 168-170) dans le milieu Liebovitz L-15 complété avec du sérum de veau à 10 %. 2 jours plus tard, le milieu est aspiré et remplacé avec du milieu L-15 frais contenant 0,8 % de carboxyméthylcellulose et 10 % de sérum de veau. On fait encore incuber ensuite les plaques à 37°C dans une atmosphère humide pendant une durée de 10 à 15 jours. Les plaques sont ensuite fixées avec du formaldéhyde (à 10 %), puis teintées, avec une solution de bleu de méthylène à 0,05 %. Dans des puits témoins, on a introduit le complément et des anticorps monoclonaux antipoliovirus neutralisants. La réaction de neutralisation a été jugée positive chaque fois que la réduction du nombre de plages observées en présence de l'anticorps, était égale ou supérieure à 50 %.

4) Immunofluorescence indirecte.

La technique utilisée a été décrite antérieurement par TARDY-PANIT et Coll.("J. Clin. Microbiol.", 1980, 11, 717-719). Le principe de la méthode était le suivant. Des cellules sont ensemencées sur des lamelles introduites dans les puits de plaques Stérilin à 25 puits et l'on ajoute ensuite une unité m.o.i. (abréviation de l'expression anglaise "multiplicity of infection"). Après des intervalles de temps déterminés à compter de l'infection, les cellules sont lavées avec une solution au chlorure de sodium tamponnée au phosphate et contenant des ions calcium et magnésium (PBS complet), séchées à l'air, fixées dans l'acétone à 0°C, puis encore séchées à l'air avant d'être mises à réagir avec les surnageants d'hybridomes. Les cellules non infectées ont été traitées de la même façon. 50 µl du surnageant de chaque hybridome testé sont placés sur chaque lamelle. Après incubation pendant 30 minutes à 37°C sous une atmosphère humide, les lamelles sont lavées trois fois à intervalles de 5 minutes avec du PBS complet, puis recouvertes avec des IgG de chèvre anti-souris (chaîne H+L de la société BEHRING), conjuguées à de l'isothocyanate de fluorescéine, puis incubées pendant une durée supplémentaire de 30 minutes. Après 3 lavages supplémentaires, les lamelles sont traitées par une solution de formaldéhyde, puis montées sur des lames dans un mélange de glycérol (à raison de 1 volume d'une solution de 10 % de formaldéhyde et de 9 volumes de glycérol) et examinées au microscope à fluorescence Lietz Dialux.

5) Marquages des anticorps monoclonaux.

Les clones (2 x $10^6$ cellules) sont lavés une fois dans le milieu minimum essentiel de Eagle ne contenant pas de méthionine (MEM-M) et remis en suspension dans 1 ml du même milieu dans les puits d'une plaque de Costar à 24 puits. On ajoute ensuite dans chacun des puits 25 µCi soit de $^{35}$S-méthionine, soit de $^3$H-méthionine (activité spécifique de 1100 Ci/mM ou 75 Ci/mM respectivement). On prépare ainsi 2 puits pour chaque clone. Après 1 heure d'incubation à 37°C sous une atmosphère de $CO_2$, on ajoute du sérum de veau foetal à chaque puits pour atteindre une concentration finale de 2,5 %. L'incubation est poursuivie pendant 2 jours à 37° C, les surnageants de chaque puits étant ensuite centrifugés pendant 5 minutes à 2000 tours par minute.

6) Détection de la fixation sur la protéine A.

0,1 ml d'une suspension à 10 % de *S. Aureus* inactivée, cultivée dans les conditions décrites par KESSLER et Coll. ("J. Immunol.", 1975, 115, 1617-1624), est ajouté à chaque surnageant, les réactifs étant ensuite maintenus en contact pendant 30 minutes à la température ambiante sous agitation, dans le but d'apprécier la capacité des anticorps monoclonaux produits par les hybridomes de fixer la protéine A. Après ce temps de réaction, les bactéries sont lavées trois fois par centrifugation avec une solution NET (TRIS-HCl 0,05 M, pH 7,4 ; NaCl 0,137 M ;

12

EDTA 0,005 M ; "NP 40" 0,05 % ; azide de sodium à 0,02 %)
contenant LiCl 0,5 M. Les immunoglobulines adhérentes
sont ensuite détachées par addition de 50 µl d'un tampon
d'électrophorèse (Tris-HCl 0,6      25 M, pH 6,8 ;
β-mercapto-éthanol   5 % ; "SDS"   2 % ; glycérol   15 %;
bleu de bromophénol 0,001 %) et par chauffage à 100°C
pendant 5 minutes. Les préparations sont ensuite soumises
à électrophorèse dans des gels de polyacrylamide-SDS à
10 % de 0,75 mm d'épaisseur pendant 3,5 heures sous
30 mA/gel en présence d'un système de refroidissement.
Les gels sont teintés avec du bleu de Coomassie brillant,
puis décolorés, séchés. La fluorographie est ensuite conduite selon la méthode de BONNAR et Coll. ("Eur. J.
Biochem.", 1974, 46, 83-88). Les gels sont ensuite exposés à un film aux rayons X de KODAK, variété X-Omat.

Les essais décrits sous les paragraphes 5)
et 6) étaient plus particulièrement destinés à la reconnaissance des anticorps monoclonaux qui, outre les capacités qu'ils possèdent de neutraliser le virus entier
et de produire une réaction décelable par immunofluorescence avec des cellules humaines en cultures préalablement infectées avec HCMV, sont en outre susceptibles
d'être précipités par la protéine A.

S'agissant de détecter parmi ces anticorps
monoclonaux ceux qui sont en outre capables de ne réagir
qu'avec un seul polypeptide de poids moléculaire de
l'ordre de 94.000, on aura recours de préférence au
marquage radioactif  ou analogue préalable des cellules
humaines préalablement infectées et dont seront ultérieurement récupérées les fractions cytoplasmiques. Ce sont
ces fractions ou extraits cytoplasmiques que l'on fera
réagir ensuite avec les anticorps monoclonaux non marqués à sélectionner.

7) Marquage radioactif des cellules destinées à fournir
   les fractions cytoplasmiques qui seront mises à réagir avec les anticorps monoclonaux à sélectionner.

13

Des cellules infectées et des cellules témoins sont ensemencées dans un milieu de culture approprié au même instant et sous les mêmes densités. Les cellules sont infectées par absorption de virus (1-2 pfu/cellule) pendant 2 heures à 37°C. Les cellules infectées sont ensuite marquées pendant 24 heures à partir du troisième jour à compter de l'instant de l'infection, après avoir été lavées une fois avec le milieu minimum essentiel dépourvu de méthionine (MEM-M) contenant de la tricine et des antibiotiques. On ajoute ensuite à chacune des cultures (dans un flacon Falcon de 175 cm$^2$) un milieu constitué par un mélange d'une partie de MEM-M pour neuf parties du milieu de base de Eagle, complété par du sérum de veau 5 % et 10 µCi/ml de $^{35}$S-méthionine (activité spécifique 1200 Ci/mM, Amersham). On soumet les flacons de culture à une rotation lente pendant la totalité de la période de marquage. Les cellules non infectées sont traitées en parallèle dans des conditions semblables.

8) Préparation des fractions cytoplasmiques.

A la fin de l'opération de marquage, les cellules sont lavées deux fois *in situ* avec du PBS complet contenant des inhibiteurs d'enzymes protéolytiques constituées par le fluorure de phényl-méthyl-sulfonyle (PSMF) (10$^{-4}$M) et par le di-isopropyl-fluoro-phosphate (DFP) (10$^{-4}$M). Les cellules sont ensuite récupérées par un grattage au sein du PBS complet contenant les inhibiteurs, la suspension étant ensuite centrifugée et remise en suspension dans le milieu RBS (MICHELSON et Coll., "J. Virol.", 32: 259-267) contenant les mêmes inhibiteurs d'enzymes protéolytiques. On laisse les cellules gonfler pendant 10 minutes sur de la glace et on ajoute le détergent commercialisé sous la désignation Nonidet-40 (NP-40) à concentration finale de 0,35 %. Les cellules sont soumises à des opérations de compression-détente alternatives à l'aide d'un piston pour rompre les parois cellulaires et les noyaux sont éliminés par centrifugation à 800 g pendant 5 minutes. Le surnageant cytoplasmique obtenu est ensuite traité avec une solution du détergent TRITON X-100 à 4 % à la température ambiante pendant 1 heure. On clarifie ensuite la solution à 15.000 g. C'est le surnageant qui constitue alors la source d'antigènes à tester.

9) <u>Immunoprécipitation des polypeptides cytoplasmiques par les anticorps monoclonaux marqués</u>.

Le surnageant susdit est réparti en plusieurs parties aliquotes ou échantillons. On procède à une pré-précipitation des protéines non spécifiques précipitables avec *S. Aureus* (0,1 ml d'une suspension de bactéries à 10 % par ml d'extrait cytoplasmique) pendant 30 minutes à la température ordinaire et sous agitation. Le *S. Aureus* est éliminé par centrifugation. On ajoute ensuite respectivement aux divers échantillons (contenant chacun la même quantité de radioactivité) :

1. Rien (à noter cependant que cet échantillon contient déjà une teneur en NP-40, ajoutée au moment de l'extraction).

2. 0,1 % de déoxycholate de sodium.

3. 0,5 % de TRITON X-100.

4. 0,1 % de SDS.

5. 0,1 % de SDS + 130 mM de β-mercapto-éthanol.

6. 130 mM de β-mercapto-éthanol.

On ajoute ensuite à chacun des échantillons l'anticorps monoclonal à étudier, puis 1 heure 30 plus tard  *S. Aureus* (75 μl d'une suspension à 10 % de bactéries) pour capter les complexes anticorps-antigènes formés. On agite pendant 10 minutes et on centrifuge la préparation. Les bactéries retenant les complexes immuns sont lavés trois fois avec la solution NET contenant LiCl 0,5 M. On procède ensuite au détachement de l'immun-complexe et à la dissociation de celui-ci par chauffage pendant 5 minutes à 100°C, au sein du susdit tampon d'électrophorèse.

La solution est ensuite passée sur un gel de polyacrylamide pour séparer les polypeptides selon leur

15

poids moléculaire.

Il est entendu que dans la technqiue qui précède, le poids moléculaire moyen de 94.000 qui a été indiqué est en correspondance avec la migration dans le gel de polyacrylamide du polypeptide marqué, obtenu à l'issue de la dissociation du susdit complexe et par référence aux distances de migration dans le même système de quatre substances ayant des poids moléculaires déterminés, à savoir la phosphorylase A de poids moléculaire 94.000, une albumine de sérum bovin de poids moléculaire 68.000, la fumérase de poids moléculaire 49.000 et l'aldolase de poids moléculaire 40.000. Par poids moléculaire 94.000, on peut entendre 94.000 $\pm$ 9.400.

Les tests qui ont été décrits ont permis d'obtenir et de sélectionner les hybridomes producteurs d'anticorps monoclonaux répondant aux caractéristiques sus-indiquées.

Dans un premier temps, les susdits tests de sélection (sous 2) ), de neutralisation (sous 3) ) et d'immunofluorescence indirecte (sous 4) ) ont permis une première sélection d'hybridomes réagissant positivement dans les tests de neutralisation avec plusieurs souches de virus HCMV. Les tests d'immunofluorescence ont été réalisés tant au niveau des membranes que du cytoplasme. Les tests de précipitation par la protéine A de *S.Aureus* ont également été effectués. Les résultats figurent dans le tableau qui suit. Dans la colonne de gauche ont été identifiés les anticorps monoclonaux en question, par référence aux hybridomes sélectionnés.

## TABLEAU

| Anticorps monoclonal N° | Neutralisation de Ad-169 | Towne | Davis | Simian virus (réduction en % du nombre de plages) | Instant auquel l'immunofluorescence a été observée pour la première fois, après l'infection, au niveau des membranes | du cytoplasme | Précipitation par S. Aureus |
|---|---|---|---|---|---|---|---|
| 57/10 | 82 | 93 | 80 | 0 | 24 h | 6 h | oui |
| 57/11 | 80 | 92 | 97 | 0 | 24 h | 15 h | non testé |
| 57/18 | 89 | 91 | 85 | 0 | 48 h | 15 h | oui |
| 57/19 | 80 | 84 | 69 | 0 | 15 h | 15 h | oui |

Les anticorps monoclonaux formés par l'ensemble de ces hybridomes se sont révélés ne pas réagir de façon non spécifique avec des récepteurs d'anti-IgG cytoplasmiques induits par HCMV.

Dans le test de sélection décrit aux paragraphes 7), 8) et 9) susdits, l'anticorps monoclonal 57/19 s'est révélé ne réagir qu'avec un polypeptide, protéine ou glycoprotéine d'un poids moléculaire de l'ordre de 94.000.

L'anticorps monoclonal selon l'invention (dans l'exemple considéré 57/19) correspond à celui qui conduit à des bandes de gel de polyacrylamide comportant pour chacun des échantillons traités une bande radioactive marquée unique au niveau du polypeptide de 94.000 daltons. Des essais en parallèle effectués en présence d'un anticorps monoclonal distinct, par exemple un anticorps monoclonal contre le virus de la poliomyélite, doivent conduire à un gel de polyacrylamide ne retenant essentiellement pas de polypeptides marqués.

Les résultats qui précèdent ont été obtenus avec l'anticorps monoclonal 57/19 qui avait été présélectionné. A l'inverse, cet essai peut être utilisé pour l'identification d'anticorps monoclonaux conformes à l'invention. Tout anticorps monoclonal qui, dans le test précédent, conduirait après dissociation de l'immun-complexe à la formation dans le gel de polyacrylamide de plusieurs bandes marquées, devrait être éliminé en tant qu'il ne permettrait pas de reconnaître et de permettre l'isolement de peptides portant un épitope séquentiel continu spécifique de HCMV.

Comme on l'a indiqué ci-dessus, les anticorps monoclonaux selon l'invention sont avantageusement applicables à des tests de diagnostic sur des biopsies tissulaires suspectées d'une infection par HCMV. Ils sont d'un intérêt tout particulier dans les essais de diagnostic, lorsqu'a priori on s'attend à des difficultés de discrimination entre les réactions antigène-anticorps spécifiques à l'égard de HCMV et les réaction non spécifiques, par réaction de l'anticorps monoclonal avec des récepteurs IgG non spécifiques.

L'invention concerne enfin un procédé pour la détection ou le diagnostic in vitro de l'infection chez l'homme par le cytomégalovirus , ce procédé consistant dans la mise en contact de cellules préalablement fixées et provenant du sujet, notamment de cellules provenant d'une biopsie tissulaire avec des anticorps monoclonaux tels que ci-dessus définis et dans la détection par immunofluorescence d'une réaction entre lesdits anticorps monoclonaux et lesdites cellules en cas d'infection.

REVENDICATIONS

1. Anticorps monoclonal-anticytomégalovirus humain, caractérisé par ses capacités :
- à neutraliser les virus de HCMV ;
- à donner lieu à des réactions détectables par immunofluorescence, sur des cellules de cultures d'origine humaine préalablement infectées avec HCMV et fixées à l'acétone ;
- à réagir avec essentiellement un seul polypeptide viral d'un poids moléculaire de l'ordre de 94.000, porteur d'un épitope séquentiel continu et susceptible d'être isolé à partir du cytoplasme de cellules d'origine humaine préalablement infectées par HCMV en la présence d'inhibiteurs de protéase ;
- de préférence, à réagir avec la protéine A.

2. Anticorps monoclonal selon la revendication 1, caractérisé en ce qu'il ne réagit pas avec des récepteurs non spécifiques d'IgG.

3. Anticorps monoclonal selon la revendication 1 ou la revendication 2, caractérisé par sa capacité à neutraliser des souches de HCMV Ad-169 Towne et Davis.

4. Anticorps monoclonal selon l'une quelconque des revendications 1 à 3, caractérisé en ce qu'il est produit par l'hybridome déposé à la C.N.C.M. sous le n° I-225 le 31/03/1983.

5. Hybridome producteur de l'anticorps selon l'une quelconque des revendications 1 à 4.

6. Procédé d'obtention et d'isolement d'hybri- domes secréteurs d'anticorps monoclonaux anti-HCMV selon l'une quelconque des revendications 1 à 4 mettant en jeu des hybridomes secréteurs d'anticorps monoclonaux capa- bles de neutraliser les virus HCMV, et préalablement formés entre des cellules de myélome et des cellules spléniques d'un animal préalablement immunisé contre un HCMV, notamment de la souche HCMV Ad-169, caractérisé en ce que :

- l'on fait réagir lesdits anticorps neutralisants avec des cellules humaines en cultures préalablement infectées avec un HCMV et préalablement fixées avec de l'acétone, puis séchées, de préférence à l'air,

- l'on effectue une première sélection de ceux des clones qui donnent lieu à une réaction de fixation, détectable par immunofluorescence sur lesdites cellules humaines après fixation de ces dernières à l'acétone et

- le cas échéant, on effectue une seconde sélection de ceux des anticorps monoclonaux retenus à l'issue de la première sélection, qui réagissent sélectivement avec une protéine virale induite dans le cytoplame desdites cellules infectées et obtenue à partir de celui-ci, cette protéine présentant un poids moléculaire de l'ordre de 94.000, ou contenue dans une fraction polypeptidique obtenue à partir dudit cytoplasme après séparation préalable des protéines ou polypeptides ayant des poids moléculaires différents et

- l'on récupère l'hybridome sélectionné à partir des produits de réaction obtenus.

7. Polypeptide ayant un poids moléculaire pouvant atteindre une valeur de l'ordre de 94.000, porteur de l'épitope séquentiel contenu dans la protéine virale cytoplasmique, susceptible d'être obtenue à partir d'une culture de cellules humaines en culture, notamment de fibroblastes humains de poumon prélablement infectés avec une culture de HCMV, notamment de la souche AD-169.

8. Polypeptide caractérisé en ce qu'il est constitué de la protéine virale cytoplasmique selon la revendication 7, sensiblement intacte.

9. Polypeptide selon la revendication 7 ou la revendication 6, caractérisé par sa capacité à réagir avec un anticorps monoclonal conforme à l'une quelconque des revendication 1 à 3.

10 - Procédé pour la détection ou le diagnostic in vitro de l'infection chez l'homme par le cytomégalovirus, caractérisé en ce qu'il comprend la mise en contact de cellules préalablement fixées et provenant du sujet, notamment de cellules provenant d'une biopsie tissulaire avec des anticorps monoclonaux tels que ci-dessus définis, et la détection par immunofluorescence d'une réaction entre lesdits anticorps monoclonaux et lesdites cellules en cas d'infection.

**0122841**

Numéro de la demande

EP 84 40 0648

Office européen
des brevets

**RAPPORT DE RECHERCHE EUROPEENNE**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| D,A | INFECTION AND IMMUNITY, vol. 36, no. 3, juin 1982, pages 924-932; L. PEREIRA et al.: "Monoclonal antibodies to human cytomegalovirus: three surface membrane proteins with unique immunological and electrophoretic properties specify cross-reactive determinants" * Page 925, colonne de gauche, ligne 38 - colonne de droite, ligne 10; page 926, lignes 10-13, 33-35; page 928, colonne de droite, lignes 3-8; page 930, tableau 4 * | 1-10 | C 12 P 21/00 C 12 N 5/00 C 12 N 15/00 G 01 N 33/58 |
| | --- | | |
| D,A | INFECTION AND IMMUNITY, vol. 38, no. 1, octobre 1982, pages 273-281, American Society for Microbiology; L.C. GOLDSTEIN et al.: "Monoclonal antibodies to cytomegalovirus: rapid identification of clinical isolates and preliminary use in diagnosis of cytomegalovirus pneumonia" * Page 274, colonne de gauche, ligne 14 - colonne de droite, ligne 49 * | 1-10 | |
| | --- | -/- | |

**DOMAINES TECHNIQUES RECHERCHES (Int. Cl. ³)**

C 12 P
C 12 N
G 01 N
A 61 K

Le présent rapport de recherche a été établi pour toutes les revendications

| Lieu de la recherche | Date d'achèvement de la recherche | Examinateur |
|---|---|---|
| LA HAYE | 09-07-1984 | RYCKEBOSCH A.O.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

\& : membre de la même famille, document correspondant

OEB Form 1503. 03.82

Office européen
des brevets

**RAPPORT DE RECHERCHE EUROPEENNE**

EP 84 40 0648

| | DOCUMENTS CONSIDERES COMME PERTINENTS | | Page 2 |

| Catégorie | Citation du document avec indication, en cas de besoin, des parties pertinentes | Revendication concernée | CLASSEMENT DE LA DEMANDE (Int. Cl. ³) |
|---|---|---|---|
| P,X | ANNALES DE VIROLOGIE, vol. 134, no. 2, 1983, pages 165-180, Paris, FR; C. AMADEI et al.: "Kinetic study of the development and localization of human cytomegalovirus-induced antigens using monoclonal antibodies" <br> * Pages 166-167, "Materials and methods"; page 169, tableau 1; page 177, lignes 18-19, 34-36; page 177, ligne 43 - page 178, ligne 4; page 178, lignes 22-26, 30-34 * <br><br> ----- | 1-10 | |
| | | | **DOMAINES TECHNIQUES RECHERCHES (Int. Cl. ³)** |

Le présent rapport de recherche a été établi pour toutes les revendications

| Lieu de la recherche <br> LA HAYE | Date d'achèvement de la recherche <br> 09-07-1984 | Examinateur <br> RYCKEBOSCH A.O.A. |
|---|---|---|